(19) Europäisches Patentamt
European Patent Office
Office européen des brevets

(11) Publication number: 0 156 508 B1

(12) # EUROPEAN PATENT SPECIFICATION

(45) Date of publication of patent specification: 28.06.89

(51) Int. Cl.[4]: A 61 K 31/415, A 61 K 9/12, A 61 K 47/00

(21) Application number: 85301187.2

(22) Date of filing: 22.02.85

(54) **Antifungal aerosol solution.**

(30) Priority: 23.02.84 US 583079

(43) Date of publication of application: 02.10.85 Bulletin 85/40

(45) Publication of the grant of the patent: 28.06.89 Bulletin 89/26

(84) Designated Contracting States: AT BE CH DE FR GB IT LI LU NL SE

(56) References cited:
EP-A-0 055 396
EP-A-0 055 397
EP-A-0 085 843
US-A-3 717 655

(73) Proprietor: ORTHO PHARMACEUTICAL CORPORATION
U.S. Route 202 P.O. Box 300
Raritan New Jersey 08869-0602 (US)

(72) Inventor: Das, Sudeb
15 Lincoln Lane
Dayton New Jersey 08810 (US)
Inventor: Martin St. FLACKS
277, Gemini Drive 2B
Somerville N.Y. 08876 (US)

(74) Representative: Jones, Alan John et al
CARPMAELS & RANSFORD 43 Bloomsbury Square
London, WC1A 2RA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 156 508 B1

**Description**

This invention relates to antifungal solutions for dermatological use and more particularly to aerosol solutions of certain imidazole derivatives which are effective in treating fungal skin infections.

Background

Imidazole derivatives of the class 1-(β-aryl)ethylimidazole ethers useful for their antifungal properties are disclosed in US—A—3,717,655. As described in this patent, imidazole derivatives of the defined class have the formula:

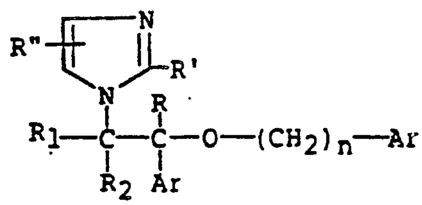

and the therapeutically active acid addition salts thereof, wherein:

R, $R_1$ and $R_2$ are each a member selected from the group consisting of hydrogen and lower alkyl;

n is the integer 1 or 2;

Ar is a member selected from the group consisting of phenyl, mono-, di- and tri-halophenyl, lower alkylphenyl, lower alkoxyphenyl, thienyl and halothienyl;

Ar' is a member selected from the group consisting of phenyl, mono, di- and tri-halophenyl, mono- and di-(lower alkyl) phenyl, lower alkoxyphenyl and cyanophenyl;

R' is a member selected from the group consisting of hydrogen, methyl and ethyl; and

R'' is a member selected from the group consisting of hydrogen and methyl.

The US—A—3 717 655 (=US—patent) further discloses that compositions comprising such imidazole derivatives as the active ingredient in a solvent or a solid, semi-solid or liquid diluent or carrier provide an effective method of combatting fungus growth. According to the patent, "The subject compounds can be used in suitable solvents or diluents, in the form of emulsions, suspensions, dispersions or ointment, on suitable solid or semi-solid carrier substances, in ordinary or synthetic soaps detergents or dispersion media, if desired, together with other compounds having arachnicidal, insecticidal, ovicidal, fungicidal and/or bactericidal effects, or together with inactive additives."

"Inert solvents used for the production of liquid preparations should preferably not be readily inflammable and should be as far as possible odorless and as far as possible non-toxic to warm-blooded animals or plants in the relevant surroundings. Solvents suitable for this purpose are high-boiling oils, for example, of vegetable origin, and lower-boiling solvents with a flash point of at least 30°C, such as, for example, isopropanol dimethysulfoxide, hydrogenated naphthalenes and alkylated naphthalenes. It is, of course, also possible to use mixtures of solvents. Solutions can be prepared in the usual way, if necessary with assistance of solution promoters. Other liquid forms which can be used consist of emulsions or suspensions of the active compound in water or suitable inert solvents, or also concentrates for preparing such emulsions, which can be directly adjusted to the required concentration. For this purpose, the active ingredient is, for example, mixed with a dispersing or emulsifying agent. The active component can also be dissolved or dispersed in a suitable inert solvent and mixed simultaneously or subsequently with a dispersing or emulsifying agent."

"Furthermore, it is possible for the active component to be used in the form of aerosols. For this purpose, the active component is dissolved or dispersed, if necessary, with the aid of suitable inert solvents as carrier liquids, such as difluorodichloromethane, which at atmospheric pressure boils at a temperature lower than room temperature, or in other volatile solvents. In this way, solutions under pressure are obtained which, when sprayed, yield aerosols which are particularly suitable for controlling or combatting fungi and bacteria, e.g., in closed chambers and storage rooms, and for application to vegetation or for eradicating or for preventing infections by fungi or bacteria."

"The subject compounds and compositions thereof can be applied by conventional methods. For example a fungus or bacterial growth or a material to be treated or to be protected against attack by fungus or bacterium can be treated with the subject compounds and the compositions thereof by dusting, sprinkling, spraying, brushing, dipping, smearing, impregnating or other suitable means."

"When the subject compounds are employed in combination with suitable carriers, e.g., in solution suspension, dust, powder, ointment or emulsion, a high activity over a very high range of dilution is observed. For example, concentrations of the active ingredient ranging from 0.1—10 percent by weight, based on the weight of composition employed, have been found effective in combatting fungi or bacteria. Of course, higher concentrations may also be employed as warranted by the particular situation."

An example of an imidazole solution is given in Example LX of the US—A—3 717 655 (US patent) where five parts of 1-[p-chloro-β-(2,6-dichlorobenzyloxy)phenethyl] imidazole are dissolved in 95 parts of alkylated naphthalene and used as a spray for the treatment of fungus infected subjects or on walls, floors

or other objects to prevent infection by fungi. Such solutions of the imidazole derivatives, while effective antifungal compositions, are obviously not suitable for dermatological use due to the presence of the strong organic solvent which has an irritating and defatting effect on tissue. Moreover, such solutions would suffer from the same defects if formulated with a propellant for aerosol application and would not be acceptable for dermatological use.

A preferred imidazole derivative for use in the present invention is 1-[2,4-dichloro-β-(2,4-dichlorobenzyloxy)phenethyl] imidazole nitrate which has the common name miconazole nitrate. The solubility of miconazole nitrate in most pharmaceutically acceptable solvents is too low to provide the desired concentration of at least one percent pharmaceutical active agent for use as a dermatological product. In many solvent systems, the incorporation of the propellant causes phase separation of an otherwise hemogeneous solution, making the formulation unsuitable for use as an aerosol spray.

EP—A—0 055 396 (Bayer) relates to compositions useful for improving the efficiency of azole derivatives as antifungal agents applied to the skin. The azole derivatives (which are different from the aryl ethers to which the present invention relates) are formulated in a composition containing small amounts of a spreading agent, a solubilising agent and a film forming agent in a large amount of a solvent. It is indicated that such compositions may be formulated as sprays by including therein up to 40% of a propellant.

It is accordingly an object of the present invention to provide a new aerosol system for compounds of the class 1-(β-aryl)ethyl-imidazole ethers. It is a further object to provide a new solvent and propellant system in which such imidazole ethers are present to the extent of at least one percent on a non-volatile residue basis excluding the propellant and volatile solvents. It is a yet further object of this invention to provide a dermatological antifungal aerosol composition comprising a 1-(β-aryl)ethyl-imidazole ether derivative in a pharmaceutically acceptable, single phase aerosol system. A still further object of this invention is to provide a dermatological antifungal composition comprising a therapeutically effective concentration of miconazole or miconazole nitrate in an aerosol solvent system. These and other objects of this invention will be apparent from the ensuing description and claims.

Summary

The dermatological antifungal compositions of the present invention comprise a therapeutically effective amount of at least about 1.0 percent by weight exclusive of propellant and other volatiles of an imidazole derivative of the type 1-(β-aryl)ethyl-imidazole in an aerosol system consisting of:

(i) 3 to 20% of a lower alkanol as a volatile solvent.

(ii) 5 to 25% of a non-volatile solubilizing agent comprising a fatty acid salt or an ester or amide of a fatty acid;

(iii) 0 to 5% of a polyhydric alcohol as a non-volatile cosolvent;

(iv) 0 to 0.05% of an antioxidant; and

(v) 60 to 85% of a normally gaseous hydrocarbon propellant.

In a preferred formulation, the imidazole derivative is miconazole or miconazole nitrate. Formulations containing from 1.0 to 2.5 percent by weight of miconazole/miconazole nitrate on a nonvolatile residue basis are effective antifungal compositions useful in treating skin infections, are pharmacologically acceptable for topical use and dry rapidly for the convenience of the user.

Description of Preferred Embodiments

The 1-(β-aryl)ethylimidazole ethers utilized as the active antifungal agent in the compositions of the present invention are known compounds, the preparation of which is disclosed in US—A—3,717,655. Such compounds have the formula:

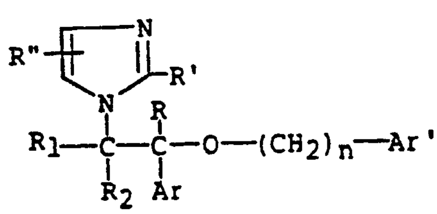

wherein R, $R_1$ and $R_2$ are each a member selected from the group consisting of hydrogen and lower alkyl;

n is the integer 1 or 2;

Ar is a member selected from the group consisting of phenyl, mono-, di- and tri-halophenyl, lower alkylphenyl, lower alkoxyphenyl, thienyl and halothienyl;

Ar′ is a member selected from the group consisting of phenyl, mono-, di- and tri-halophenyl, mono- and di-(lower alkyl) phenyl, lower alkoxyphenyl and cyanophenyl;

R′ is a member selected from the group consisting of hydrogen, methyl and ethyl; and

R″ is a member selected from the group consisting of hydrogen and methyl.

As used herein, "lower alkyl" and "lower alkoxy" may be straight or branched chained saturated hydrocarbons having from 1 to 6 carbons, such as, for example, methyl, ethyl, propyl, isopropyl, butyl,

pentyl, hexyl and the like alkyls, and, respectively, the corresponding alkoxys such as methoxy, ethoxy, propoxy, isopropoxy, etc. The preferred lower alkyl and lower alkoxy are methyl and methoxy, respectively. The term "halo" refers to halogens of atomic weight less than 127, i.e., fluoro, iodo, bromo, and chloro.

A particularly preferred imidazole ether representative of the above compounds is miconazole, 1-[2,4-dichloro-β-(2,4-dichlorobenzyloxy)phenethyl] imidazole as the base or as the nitrate salt having the formula:

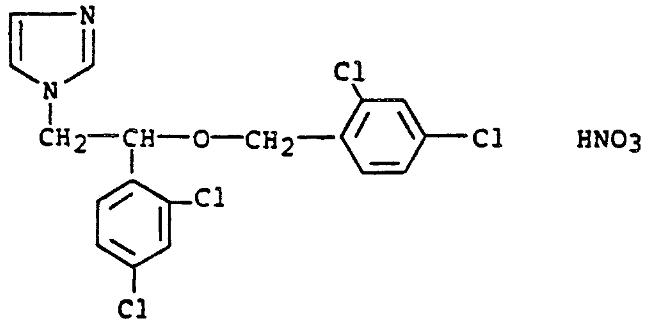

The imidazole ethers in general and the miconazoles in particular have very low solubilities in most solvents suitable for use in topical pharmaceutical compositions such as in the treatment of fungal skin infections. The solubility of miconazole nitrate, for example, in several common solvents has been determined to be as follows:

| Solvent | G/100 ml. |
|---|---|
| Water | 0.03 |
| NaOH | Insoluble |
| HCl 0.1 N | 0.05 |
| 40% PG/water | 0.13 |
| Propylene Glycol | 0.15 |
| PEG 300 | 0.13 |
| Methanol | 1.5 |
| Ethanol | 0.76 |
| 2-Propanol | 0.13 |
| Acetone | 0.34 |
| Hexane | 0.03 |
| Chloroform | 0.31 |
| Ethyl Acetate | 0.07 |
| Benzene | 0.05 |
| Methyl Isobutylketone | 0.04 |
| Tetrahydrofuran | 0.2 |

In the treatment of fungal skin infections, it is desirable that the antifungal agent be easily applied with a minimum of discomfort and inconvenience to the user. Miconazole nitrate has already found wide application as an over-the-counter antifungal preparation in the form of ointment, creams and dry powders. Attempts to formulate miconazole nitrate as an aerosol solution which could be dispensed with conventional hydrocarbon propellants was frustrated by the extremely low solubility of miconazole nitrate

in pharmaceutically acceptable solvents compatible with hydrocarbon propellants. The present invention overcomes this problem by providing a solvent system capable of dissolving from 1.0 to about 2.5 percent by weight miconazole nitrate exclusive of propellant and other volatiles to provide a single phase, quick drying and effective aerosol antifungal composition.

The solvent/propellant system according to this invention consists essentially of:

(i) 3 to 20% of a lower alkanol as a volatile solvent;

(ii) 5 to 25% of a non-volatile solubilizing agent comprising a fatty acid salt or an ester or amide of a fatty acid;

(iii) 0 to 5% of a polyhydric alcohol as a non-volatile cosolvent;

(iv) 0 to 0.05% of an antioxidant; and

(v) 60 to 85% of a normally gaseous hydrocarbon propellant.

The volatile solvent (i) is preferably ethanol or a mixture of ethanol and benzyl alcohol. The solubilizing agent (ii) is preferably selected from the group consisting of sorbitan sesquioleate, polysorbate 20, polysorbate 60, sodium laurate, sodium oleate, cocamide diethanolamide (Cocamide DEA), sodium lauryl sulfate, lauramide diethanolamide (Lauramide DEA), dioctyl sodium sulfosuccinate and mixtures thereof. The polyhydric alcohol (iii) is preferably selected from the group consisting of polyethylene glycol 200 or 400, glycerin, propylene glycol, dipropylene glycol and mixtures thereof. The antioxidant (iv) is preferably selected from the group consisting of butylated hydroxytoleune, butylated hydroxyanisole, tocopherols and mixtures thereof. The hydrocarbon propellant (v) is preferably selected from the group consisting of n-butane, iso-butane, propane, and mixtures thereof.

Specific aerosol formulations may be selected from the various suitable and preferred components as identified above or their equivalents according to the particular needs and desires of the formulator. Representative examples of some formulations provided acceptable single phase aerosol solutions for miconazole and miconazole nitrate are as follows:

| | | % w/w | % w/w basis non-volatiles |
|---|---|---|---|
| a) | Miconazole Nitrate | 0.188 | 2.14 |
| | Tocopherols | 0.003 | |
| | Benzyl Alcohol | 1.981 | |
| | Cocamide DEA | 2.368 | |
| | Sorbitan Sesquioleate | 4.239 | |
| | Ethyl Alcohol | 16.831 | |
| | Hydrocarbon Propellant A—46 qs | 100.000 | |
| b) | Miconazole Nitrate | 0.289 | 1.38 |
| | Butylated hydroxytoluene | 0.003 | |
| | Cocamide DEA | 9.378 | |
| | Sorbitan Sesquioleate | 10.806 | |
| | Polyethylene Glycol 400 | 0.375 | |
| | Ethyl Alcohol | 4.761 | |
| | Hydrocarbon Propellant qs to | 100.000 | |
| c) | Miconazole Nitrate | 0.342 | 2.0 |
| | Sorbitan Sesquioleate | 15.880 | |
| | Dipropylene Glycol | 0.854 | |
| | Ethyl Alcohol | 8.540 | |
| | Hydrocarbon Propellants qs to | 100.000 | |
| d) | Miconazole Nitrate | 0.269 | 2.1 |
| | Butylated hydroxyanisole | 0.003 | |
| | Cocamide DEA | 3.839 | |
| | Sorbitan Sesquioleate | 7.486 | |
| | Polyethylene Glycol 200 | 1.216 | |
| | Ethyl Alcohol | 12.795 | |
| | Hydrocarbon Propellants qs to | 100.000 | |
| e) | Miconazole Nitrate | 0.342 | 2.0 |
| | Lauramide DEA | 16.734 | |
| | Ethyl Alcohol | 8.540 | |
| | Hydrocarbon Propellants qs to | 100.000 | |

| | | % w/w | % w/w basis non-volatiles |
|---|---|---|---|
| f) | Miconazole Nitrate | 0.294 | 2.0 |
| | Cocamide DEA | 4.704 | |
| | Sorbitan Sesquioleate | 9.261 | |
| | Cococaprylate | 0.441 | |
| | Ethyl Alcohol | 10.900 | |
| | Hydrocarbon Propellants qs to | 100.000 | |

The above formulations are readily prepared by first mixing together the solvents and solubilizing agent to obtain a uniform clear solution. The miconazole (base or nitrate) is next dissolved in the solution with vigorous agitation. When the solution again clears, the antioxidant is added to complete the formulation of the aerosol concentrate which is then transferred to a suitable container and pressurized with the hydrocarbon propellant.

Aerosol solutions prepared in accordance with the present invention contain from 1.0 to 2.5 percent active imidazole by weight on the basis of non-volatile residue.

Such solutions of miconazole are effective antifungal dermatological compositions which are pharmacologically acceptable for topical application in the treatment of fungal skin infections.

**Claims**

1. A dermatological antifungal aerosol composition comprising:

(a) A solvent and propellant system consisitng of 3 to 20% of a lower alkanol as a volatile solvent; 5 to 25% of a non-volatile solubilizing agent comprising a fatty acid salt or an ester or amide of a fatty acid; 0 to 5% of a polyhydric alcohol as a non-volatile cosolvent; 0 to 0.05% of an antioxidant; and 60 to 85% of a normally gaseous hydrocarbon as a propellant; and

(b) a therapeutically effective amount equal to at least 1.0% by weight of the aerosol composition exclusive of propellant and other volatiles of a 1-(β-aryl)ethylimidazole derivative having the formula:

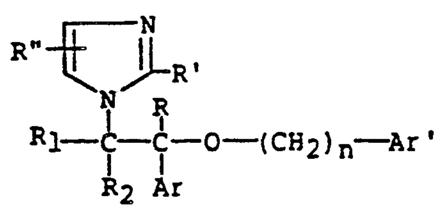

and the therapeutically active acid addition salts thereof, wherein:

R, $R_1$ and $R_2$ are each a member selected from the group consisting of hydrogen and lower alkyl;

n is the integer 1 or 2;

Ar is a member selected from the group consisting of phenyl, mono-, di- and tri-halophenyl, lower alkylphenyl, lower alkoxyphenyl, thienyl and halothienyl;

Ar' is a member selected from the group consisting of phenyl, mono, di- and tri-halophenyl, mono- and di-(lower alkyl) phenyl, lower alkoxyphenyl and cyanophenyl;

R' is a member selected from the group consisting of hydrogen, methyl and ethyl; and

R'' is a member selected from the group consisting of hydrogen and methyl.

2. A solution of Claim 1 wherein said lower alkanol is ethyl alcohol.

3. A solution of claim 1 or claim 2 wherein said solubilizing agent is selected from the group consisting of sorbitan sesquioleate, polysorbate 20, polysorbate 60, sodium laurate, sodium oleate, cocamide diethanolamide, sodium lauryl sulfate, lauramide diethanolamide, dioctyl sodium sulfosuccinate and mixtures thereof.

4. A solution of any one of claims 1 to 3 wherein said antioxidant is selected from the group consisting of butylated hydroxytoluene, butylated hydroxy anisole, tocopherols and mixtures thereof.

5. A solution of any one of claims 1 to 4 wherein said hydrocarbon propellant is selected from the group consisting of n-butane, iso-butane, propane and mixtures thereof.

6. A solution of any one of claims 1 to 5 wherein said imidazole derivative is 1-[2,4-dichlorobenzyloxy)-phenethyl]-imidazole or 1-[2,4-dichloro-β-dichlorobenzyloxy)phenethyl]-imidazole nitrate.

7. A solution of Claim 6 wherein said solvent and propellant system consists essentially of from 15 to 20% by weight of said system of a solvent comprising 15 to 20% ethyl alcohol and 0 to 3% benzyl alcohol; from 5 to 10% by weight of said system of solubilizing agent selected from the group consisting of cocamide diethanolamide, sorbitan sesquioleate, and mixtures thereof; from 0.001 to 0.005% by weight of said system of an antioxidant; and from 70 to 80% by weight of said system of a hydrocarbon propellant.

8. A solution of claim 7 wherein said antioxidant is tocopherols.

9. A method of producing an aerosol composition according to any one of claims 1 to 8 comprising mixing together to form a homogeneous solution from 3 to 20% of a lower alkanol as a volatile solvent; 5 to 25% of a non-volatile solubilizing agent comprising a fatty acid salt or an ester or amide of a fatty acid; 0 to 5% of a polyhydric alcohol as a non-volatile cosolvent; 0 to 0.5% of an antioxidant; and a therapeutically effective amount equal to at least 1% by weight of the composition exclusive of volatiles of a 1-(B-aryl) ethylimidazole as defined in claim 1 and pressurising the homogeneous solution with 60 to 85% of a normal gaseous hydrocarbon as a propellant.

**Patentansprüche:**

1. Dermatologische fungizide Aerosol-Zuammensetzung, enthaltend:

(a) ein Lösungsmittel- und Triebmittelsystem, bestehend aus 3 bis 20% eines niederen Alkanols als flüchtiges Lösungsmittel; 5 bis 25% eines nicht-flüchtigen Solubilisierungsmittels, umfassend eine Fettsäuresalz oder einen Ester oder ein Amid einer Fettsäure; 0 to 5% .eines mehrwertigen Alkohols als nicht-flüchtiges Co-Lösungsmittel; 0 bis 0,05% eines Antioxidationsmittels; und 60 bis 85% eines normalerweise gasförmigen Kohlenwasserstoffes als Treibmittel; und

(b) eine therapeutisch wirksame Menge, entsprechend wenigstens 1,0 Gew.-% der Aerosol-Zusammensetzung ausschließlich des Treibmittels und anderer flüchtiger Bestandteile, eines 1-(β-Aryl))ethylimidazolderivats mit der Formel:

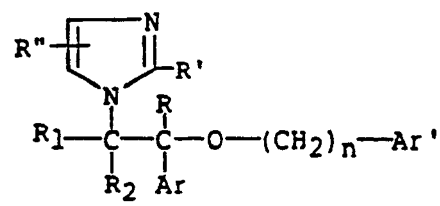

und der therapeutisch wirksamen Säureadditonssalze hievon, worin:

R, $R_1$ und $R_2$ jeweils ein Glied, ausgewählt aus der aus Wasserstoff und Niederalkyl bestehenden Gruppe, darstellen;

n die ganze Zahl 1 oder 2 bedeutet;

Ar ein Glied, ausgewählt aus der aus Phenyl, Mono-, Di- und Trihalogenphenyl, Niederalkylphenyl, Niederalkoxyphenyl, Thienyl und Halogenthienyl bestehenden Gruppe, bedeutet;

Ar′ für ein Glied, ausgewählt aus der aus Phenyl, Mono, Di- und Tri-halogenphenyl, Mono- und Di(niederalkyl)phenyl, Niederaalkoxyphenyl und Cyanophenyl bestehenden Gruppe, steht;

R′ für aus der aus Wasserstoff, Methyl und Ethyl bestehenden Gruppe ausgewähltes Glied steht; und

R″ für ein aus der aus Wasserstoff und Methyl bestehenden Gruppe ausgewähltes Glied steht.

2. Lösung nach Anspruch 1, worin das genannte niedere Alkanol Ethylalkohol ist.

3. Lösung nach Anspruch 1 oder 2, worin das genannte Solubilizierungsmittel aus der aus Sorbitansesquioleat, Polysorbat 20, Polysorbat 60, Natriumlaurat, Natriumoleat, Cocosamiddiethanolamid, Natriumlaurylsulfat, Lauramiddiethanolamid, Dioctylnatriumsulfosuccinat und Gemischen hievon bestehenden Gruppe ausgewählt ist.

4. Lösung nach einem der Ansprüche 1 bis 3, worin das genannte Antioxidationsmittel aus der aus butyliertem Hydroxytoluol, butyliertem Hydroxyanisol, Tocopherolen und Gemischen hievon bestehenden Gruppe ausgewählt ist.

5. Lösung nach einem der Ansprüche 1 bis 4, worin das genannte Kohlenwasserstofftriebmittel aus der aus n-Butan, Isobutan, Propan und Gemischen hievon bestehenden Gruppe ausgewählt ist.

6. Lösung nach einem der Ansprüche 1 bis 5, worin das genannte Imidazolderivat 1-[2,4-Dichlor-β-(2,4-dichlorbenzyloxy)phenethyl]-imidazol oder 1-[2,4-Dichlor-β-(2,4-dichlorobenzyloxy)phenethyl]-imidazolnitrat ist.

7. Lösung nach Anspruch 6, worin das genannte Lösungsmittel- und Triebmittel- system im wesentlichen aus 15 bis 20 Gew.-% dieses Systems aus einem 15 bis 20% Ethylalkohol und 0 bis 3% Benzylalkohol umfassenden Lösungsmittel: aus 5 bis 10 Gew.-% dieses Systems aus Solubilisierungsmittel, ausgewählt aus der aus Cocosamiddiethanolamid, Sorbitansesquioleat und Gemischen hievon bestehenden Gruppe; aus 0,001 bis 0,005 Gew.-% dieses Systems aus einem Antioxidationsmittel; und aus 70 bis 80 Gew.-% dieses Systems aus einem Kohlenwasserstofftreibmittel besteht.

8. Lösung nach Anspruch 7, worin das Antioxidationsmittel ein Tocopherol ist.

9. Verfahren zur Herstellung einer Aerosol-Zusammensetzung nach einem der Ansprüche 1 bis 8, umfassend das Zusammenmischen unter Ausbildung einer homogenen Lösung von 3 bis 20% eines niederen Alkanols als ein flüchtiges Lösaungsmittel; von 5 bis 25% eines nicht-flüchtigen Solubilisierungsmittels, umfassend eine Fettsäuresalz oder einen Ester oder ein Amid einer Fettsäure; von

0 bis 5% eines mehrwertigen Alkohols als ein nicht-flüchtiges Co-Lösungsmittel; von 0 bis 0,05% eines Antioxidationsmittels; und einer therapeutisch wirksamen Menge, entsprechend wenigstens 1 Gew.-% der Zusammensetzung, ausgenommen flüchtige Bestandteil, eines 1-(β-Aryl)ethylimidazols, wie in Anspruch 1 definiert, und ein Druckbeaufschlagen der homogenen Lösung mit 60 bis 85% eines normalwerweise gasförmigen Kohlenwasserstoffes als Treibmittel.

**Revendications**

1. Composition d'aérosol antifongique à usage dermatoligique comprenant:

(a) un système solvant et vecteur consistant en 3 à 20% d'un alcanol inférieur comme solvant volatil; 5 à 25% d'un agent solubilisant non volatil comprenant un sel d'acide gras ou un ester ou amide d'un acide gras; de 0 à 5% d'un alcool polyhydrique comme cosolvant non volatil; de 0 à 0,05% d'un antioxidant; et de 60 à 85% d'un hydrocarbure normalement gazeux comme vecteur; et

(b) un quantité thérapeutiquement efficace égale à au moins 1,0% en poids de la composition d'aérosol à l'exclusion du vecteur et des autres fractions volatiles d'un dérivé de 1-(β-aryl)éthyl-imidazole de formule:

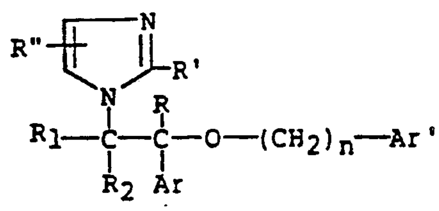

et ses sels d'addition d'acides thérapeutiquement actifs, où

R, $R_1$ et $R_2$ représentent chacun un membre choisi dans le groupe constitué par hydrogène et alcoyle inférieur;

n est le nombre entier 1 ou 2;

Ar est un membre choisi dans le groupe constitué par phényle, mono- di- et tri-halophényle, alcoyle inférieur-phényle, alcoxy inférieur-phényle, thiényle et halothiényle;

Ar' est un membre choisi dans le groupe consistué par phényle, mono-, di- et tri-halophényle, mono et di(alcoyle inférieur)phényle, alcoxy inférieur-phényle et cyano phényle;

R' est un membre choisi dans le groupe constitué par hydrogène, méthyle et éthyle; et

R'' est un membre choisi dans le groupe constitué par hydrogène et méthyle.

2. Solution de la revendication 1 où ledit alcanol inférieur est l'alcool éthylique.

3. Solution de la revendication 1 ou de la revendication 2 où ledit agent solubilisant est choisi dans le groupe constitué par le sesquioléate de sorbitan, le polysorbate 20, le polysorbate 60, le laurate de sodium, l'oléate de sodium, le diéthanolamide de cocamide, le lauryl-sulfate de sodium, le diéthanolamide de lauramide, le sulfosuccinate de dioctyl-sodium et leurs mélanges.

4. Solution de l'une quelconque des revendications 1 à 3 où ledit antioxydant est choisi dans le groupe continué par l'hydroxytoluéne butylé, l'hydroxyanisol butylé, les tocophérols et leurs mélanges.

5. Solution de l'une quelconque des revendications 1 à 4 où ledit vecteur hydrocarboné est choisi dans le groupe constitué par le n-butane, l'iso-butane, le propane et leurs mélanges.

6. Solution de l'une quelconque des revendications 1 à 5 où ledit dérivé d'imidazole est le 1-[2,4-dichloro-β-(2,4-dichlorobenzyloxy)-phénéthyl]-imidazole ou le 1-[2,4-dichloro-β-(2,4-dichlorobenzyloxy)-phénéthyl]-imidazole nitrate.

7. Solution de la revendication 6 où ledit système solvant et vecteur consiste essentiellement en 15 à 20% en poids dudit système d'un solvant comprenant de 15 à 20% d'alcool éthylique et de 0 à 3% d'alcool benzylique; de 5 à 10% en poids dudit système d'agent solubilisant choisi dans le groupe constitué par le diéthanolamide de cocamide, le sesquioléate de sorbitan, et leurs mélanges; de 0,001 à 0,005% en poids dudit système d'un antioxydant; et de 70 à 80% en poids dudit système d'un vecteur hydrocarboné.

8. Solution de la revendication 7 où ledit antioxydant est formé de tocophérols.

9. Procédé de production d'une composition d'aérosols selon l'une quelconque des revendications 1 à 8 dans lequel on mélange pour former une solution homogène de 3 à 20% d'un alcanol inférieure comme solvant volatil; de 5 à 25% d'un agent solubilisant non volatil comprenant un sel d'acide gras ou un ester ou amide d'un acide gras; de 0 à 5% d'un alcool polyhydrique comme cosolvant non volatil; de 0 à 0,05% d'un antioxydant; et une quantité thérapeutiquement efficace égale à au moins 1% en poids de la compositon non compris les fractions volatiles d'un 1-(β-aryl)éthylimidazole tel que défini dans la revendication 1 et on pressurise la solution homogène avec 60 à 85% d'un hydrocarbure gazeux normal comme vecteur.